# EUROPÄISCHE PATENTSCHRIFT

(11) **EP 1 696 809 B1**
(45) Veröffentlichungstag und Bekanntmachung des Hinweises auf die Patenterteilung: **05.08.2009**
(21) Anmeldenummer: 04797689.9
(22) Anmeldetag: 06.11.2004
(51) Int. Cl.: A61B 17/70, A61B 17/80

(54) **FEDERELEMENT FÜR EINE STABILISIERUNGSEINRICHTUNG FÜR KNOCHEN**
SPRING ELEMENT FOR A BONE STABILIZING DEVICE
ELEMENT RESSORT POUR UN DISPOSITIF DE STABILISATION POUR DES OS

(30) Priorität: 07.11.2003 DE 10351978; 07.11.2003 US 518469 P; 21.11.2003 US 523946 P; 03.03.2004 US 550182 P
(43) Veröffentlichungstag der Anmeldung: 06.09.2006
(73) Patentinhaber: BIEDERMANN MOTECH GmbH, 78054 VS-Schwenningen (DE)
(72) Erfinder: BIEDERMANN, Lutz, 78048 VS-Villingen (DE); MATTHIS, Wilfried, 79367 Weisweil (DE); HARMS, Jürgen, 76227 Karlsruhe (DE)
(74) Vertreter: Hofer, Dorothea
(86) Internationale Anmeldenummer: PCT/EP2004/012591
(87) Internationale Veröffentlichungsnummer: WO 2005/044117

(56) Entgegenhaltungen:
- EP-A- 0 677 277
- WO-A-02/07622
- WO-A-03/047442
- FR-A- 2 717 370
- US-B1- 6 267 764

## Beschreibung

Die Erfindung betrifft ein Federelement für die Verwendung in der Wirbelsäulen- oder Unfallchirurgie gemäß dem Oberbegriffs des Patentanspruchs 1.

Zur Fixierung von Knochenfrakturen oder zur Stabilisierung der Wirbelsäule sind Fixations- und Stabilisierungseinrichtungen bekannt, die aus wenigstens zwei im Knochen bzw. Wirbel verankerten und über eine Platte oder über einem Stab verbundenen Knochenschrauben bestehen. Derartige starre Systeme erlauben keine Bewegung der relativ zu einander fixierten Knochenteile oder Wirbel.

Für bestimmte Indikationen ist jedoch eine dynamische Stabilisierung wünschenswert, bei der die zu stabilisierenden Knochenteile und Wirbel eine kontrollierte begrenzte Bewegung zu einander ausführen können. Eine Möglichkeit für die Realisiering der dynamischen Stabilisierungseinrichtung besteht in der Verwendung eines elastischen Elementes anstelle eines die Knochenverankerungselemente verbindenden starren Stabes.

Aus der US 2003/0109880 A1 ist eine dynamische Stabilisierungseinrichtung für Wirbel bekannt, die eine erste und eine zweite im Wirbel zu verankernde Schraube jeweils mit einem Aufnahmeteil zum Einlegen einer die Schrauben verbindenden Feder und eine solche Feder umfaßt. Die Feder selbst ist als Ganzes in Form einer Schraubenfeder mit dicht benachbarten Windungen nach Art einer Zugfeder ausgebildet und wird über Klemmschrauben in den Aufnahmeteilen fixiert. Es besteht hierbei jedoch die Gefahr, daß die Feder auf Grund ihrer Elastizität dem Druck der Klemmschraube ausweicht und somit die Fixierung zwischen der Knochenschraube und der Feder gelockert wird. Ein weiterer Nachteil der Vorrichtung besteht darin, daß die Elastizität der Feder bei ansonsten gleichen Federeigen-schaften von der Länge der Feder abhängt.

Aus der EP 0 669 109 B1 ist eine Stabilisierungseinrichtung zum Stabilisieren benachbarter Rückenwirbel bekannt, die zwei monoaxiale Pedikelschrauben und ein Band umfaßt, welches in den Aufnahmeteilen der Pedikelschrauben jeweils über eine Klemmschraube befestigt ist und welche ein auf das Band aufgezogenes druckfestes Stützelement beinhaltet. Mit einer derartigen Stabilisierungseinrichtung wird jedoch keine Stabilisierung gegen Torsion erreicht. Wie bei der weiter oben beschriebenen Stabilisierungseinrichtung ist die Elastizität der Verbindung der beiden Knochenverankerungselemente bei ansonsten gleichen Eigenschaften der Federelemente abhängig von der Länge der Federelemente bzw. von dem Abstand der Knochenverankerungselemente.

Aus der US 6,162,223 ist eine Fixationseinrichtung für ein Gelenk, z.B. für ein Handgelenk oder ein Kniegelenk, bekannt, bei der ein an seinen Enden mit Knochenverankerungselementen verbundener Fixationsstab zweiteilig ausgebildet ist, wobei die zwei Teile des Fixationsstabs über ein flexibles Kupplungsteil miteinander verbunden sind und wobei die Fixationsstäbe um das Kupplungsteil außerhalb des Körpers angebracht sind. Die beiden Teile des Fixationsstabs sind mit dem Kupplungsteil nicht fest verbunden, sondern können sich entlang einer Bohrung in dem Kupplungsteil frei bewegen. Der Durchmesser des Kupplungsteil ist auf Grund der Art der Verbindung mit dem zweiteiligen Fixationsstab immer größer als der Durchmesser des Fixationsstabs. Diese bekannte Fixationseinrichtung ist auf Grund ihres komplizierten und voluminösen Aufbaus zum inneren Einsatz an der Wirbelsäule oder anderen Knochen nicht geeignet.

Aus der WO 03/047442 A1 ist ein elastisches Element zur Verwendung in einer Stabilisierungseinrichtung für Knochen oder Wirbel bekannt, das als ein im Wesentlichen zylindrischer Körper mit einem ersten Ende und einem diesem gegenüberliegenden zweiten Ende und einer sich von dem ersten zum zweiten Ende erstreckenden koaxialen Bohrung ausgebildet ist, der an seinen gegenüberliegenden Enden ein Innengewinde zum Verbinden mit einem Schaft und/oder einem Kopf einer Knochenschraube oder zum Verbinden mit einem Stababschnitt oder einer Platte aufweist, wobei in der Bohrung ein Kern vorgesehen ist. Der Kern wird durch ein Federelement aus einem Polymer gebildet.

In dem zylindrischen Körper ist eine spiralförmige Ausnehmung mit über deren Länge hinweg konstanter Breite gebildet. Für die beiden Enden der spiralförmigen Ausnehmung sind keine gesonderten Massnahmen getroffen.

Es ist Aufgabe der Erfindung, ein Federelement bereit zu stellen, das einfach und kompakt gebaut, sowie leicht zu handhaben bei gleichzeitig hoher Sicherheit im Einsatz ist, und welches mit anderen Elementen in möglichst vielfältiger Weise zu einer dynamischen Stabilisierungseinrichtung für miteinander zu verbindende Wirbel oder Knochen kombiniert werden kann.

Die Aufgabe wird gelöst durch ein Federelement nach dem Patentanspruch 1. Weiterbildungen der Erfindung sind in den Unteransprüchen angegeben.

Die Erfindung weist einen Vorteil auf, daß die am Auslauf einer spiralförmigen Ausnehmung entstehenden Belastungsspitzen in dem Material des Elementkörpers aufgrund des als Viertelkreis ausgebildeten Auslaufs gering gehalten werden.

Das Federelement ist wahlweise mit starren stabförmigen Elementen verschiedener Länge zu einem elastischen stabförmigen Element kombinierbar ist oder mit verschiedenen Schäften und/oder Köpfen zu einer Knochenschraube mit elastischen Eigenschaften kombinierbar ist. Der Federstab bzw. die Knochenschraube weisen dann in Abhängigkeit von dem verwendetem Federelement vorgegebene elastische Eigenschaften wie eine bestimmte Kompressions- und Extensionsfähigkeit in axialer Richtung, sowie eine bestimmte Biege- und Torsionssteifigkeit auf.

Insbesondere kann das Federelement mit stabförmigen Bauteilen verschiedener Dicke oder mit in der Wirbelsäulen- und/oder Unfallchirurgie zu verwendenden Platten unterschiedlicher Form und Länge verbunden werden.

Weitere Merkmale und Zweckmäßigkeiten ergeben sich aus der Beschreibung von Ausführungsbeispielen anhand der Figuren.

Von den Figuren zeigen:
- Fig. 1a: eine Seitenansicht eines ersten Federelementes;
- Fig. 1b: eine Schnittdarstellung des Federelementes aus Fig. 1a;
- Fig. 2a: eine erste Anwendung des Federelements in Form eines stabförmigen Elements;
- Fig. 2b: eine Abwandlung von Fig. 2a;
- Fig. 3a: ein Knochenverankerungselement mit dem Federelement aus Fig. 1a;
- Fig. 3b: eine Schnittdarstellung eines Teils des Elements von Fig. 3a mit einer Weiterbildung;
- Fig. 3c: eine Schnittdarstellung des Teils von Fig. 3b entlang der Linie A-A;
- Fig. 4: eine Stabilisierungseinrichtung bestehend aus zwei dreiteiligen Knochenverankerungselementen und einem stabförmigen Element, die jeweils ein Federelement aufweisen;
- Fig. 5: eine Seitenansicht eines zweiten Federelementes;
- Fig. 6: eine Seitenansicht eines dritten Federelementes;
- Fig. 7a: eine Seitenansicht eines vierten Federelementes;
- Fig. 7b: eine um 90° gedrehte Seitenansicht des Federelementes aus Fig. 4a;
- fig. 8: eine Schnittdarstellung eines fünften;
- Fig. 9.: ein sechstes Federelement;
- Fig. 10: ein siebtes Federelement;
- Fig. 11a: eine Explosionsdarstellung eines Verbindungselementes, das aus einem stabförmigen Element, einem Federelement und einer Platte besteht;
- Fig. 11b: eine Schnittdarstellung der Platte aus Fig. 11a entlang der Linie A-A;
- Fig. 12: eine Anwendung der Platte aus Fig. 11a und 11b, bei dem die Platte und das mit der Platte über ein Federelement verbundene stabförmige Element mit Knochenverankerungselementen jeweils in Wirbeln verankert ist;
- Fig. 13a: eine Anwendung des Federelementes in einer dynamischen Stabilisierungseinrichtung für einen Beckenknochen;
- Fig. 13b: eine Schnittdarstellung eines bei der Stabilisierungseinrichtung aus Fig. 13a verwendeten Knochenverankerungselementes;
- Fig. 14: eine Anwendung des Federelementes in einem Fixateur Externe mit Schanzschrauben, die über stabförmige Elemente miteinander verbunden sind; und
- Fig. 15: ein mittels Drahterodieren, Laserbearbeitung oder Wasserstrahlbearbeitung hergestelltes erfindungsgemäßes Federelement.

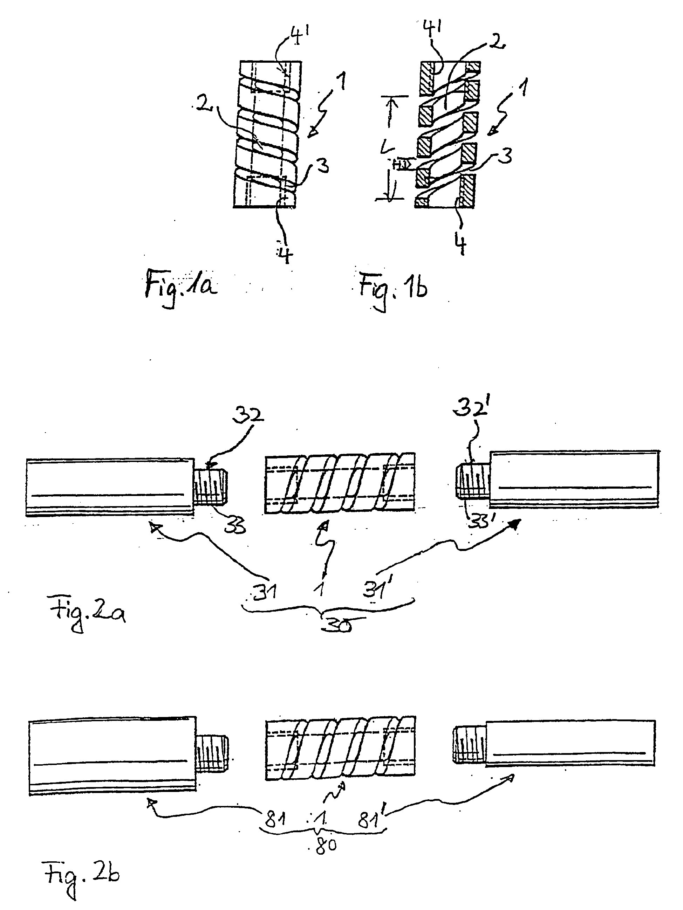

Die Figuren 1a - 14 dienen lediglich der allgemeinen technischen Erläuterung und zeigen nicht den Teilgegenstand der Erfindung, wie er im kennzeichnenden Teil des Hauptanspruchs angegeben ist.

In den Figuren 1a und 1b ist ein erstes Federelement 1 dargestellt.

Das Federelement 1 besteht aus einem zylindrischen Rohr mit einer durchgehenden koaxialen Bohrung 2 und einer spiralförmig mit einer vorbestimmten Steigung und über eine vorbestimmte Länge in Richtung der Zylinderachse in der Wandung verlaufenden Ausnehmung 3, die in radialer Richtung in die Bohrung 2 mündet. Dadurch ist eine Schraubenfeder gebildet. Die Länge der spiralförmigen Ausnehmung in Richtung der Zylinderachse, die Höhe der Ausnehmung, die Steigung der Spirale und der Durchmesser der koaxialen Bohrung sind so gewählt, daß eine gewünschte Steifigkeit der Schraubenfeder gegenüber axialen Kräften, Biegekräften und Torsionskräften, die auf das Federelement wirken, gegeben ist. Angrenzend an seine beiden freien Enden weist das Federelement 1 jeweils ein sich über eine vorbestimmte Länge erstreckendes Innengewinde 4, 4' auf. Der Außendurchmesser des Federelementes ist der jeweiligen Anwendung entsprechend gewählt.

In einer ersten, in Fig. 2a gezeigten Anwendung ist das Federelement 1 Bestandteil eines elastischen stabförmigen Elements 30. Das elastische stabförmige Element 30 besteht aus dem Federelement 1 und zwei zylindrischen Stabschnitten 31, 31', die an ihrem Ende jeweils einen zylindrischen Ansatz 32, 32' mit einem Außengewinde 33, 33' aufweisen, das mit dem Innengewinde 4, 4' des Federelements 1 zusammenwirkt. Die Stababschnitte und das Federelement weisen in diesem Ausführungsbeispiel im wesentlichen denselben Außendurchmesser auf. Die Länge der Stababschnitte 31, 31' und des Federelements 1 sind unabhängig voneinander im Hinblick auf eine gewünschte Anwendung wählbar. Das stabförmige Element dient beispielsweise zur Verbindung von Pedikelschrauben an der Wirbelsäule. Das so gebildete stabförmige Element 30 nimmt durch die elastischen Eigenschaften des Federelementes 1 in vorgegebenen Umfang Kompressions-, Extensions-, Biege- und Torsionskräfte auf.

In Fig. 2b ist ein elastisches stabförmiges Element 80 gezeigt, das sich von dem elastischen stabförmigen Element 30 dadurch unterscheidet, daß ein erster starrer Stababschnitt 81 einen größeren Außendurchmesser aufweist als das Federelement 1 und der zweite starre Stababschnitt 81' einen kleineren Außendurchmesser aufweist, als das Federelement 1. Alternativ können auch beide Stababschnitte einen größeren oder einen kleineren Durchmesser haben als das Federelement.

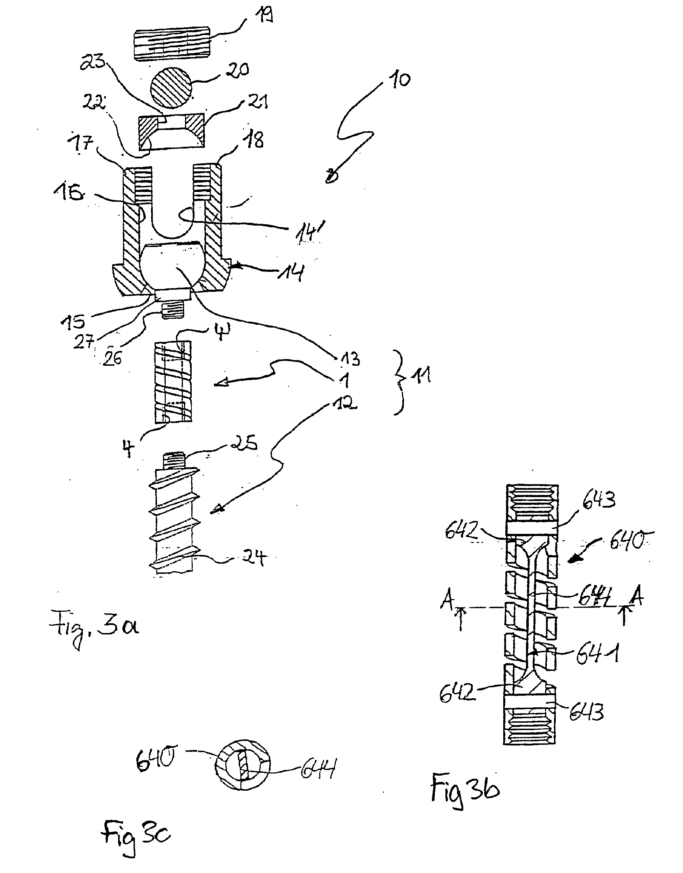

Fig. 3a zeigt eine zweite Anwendung des Federelementes. Das Federelement 1 ist hier Bestandteil eines Knochenverankerungselements 10, das als Polyaxial-knochenschraube ausgebildet ist. Die Polyaxialknochenschraube weist ein Schraubenelement 11 auf, welches aus dem Federelement 1, einem Schaft 12 mit einer nicht dargestellten Spitze und einem Schraubenkopf 13 besteht.

Der Schaft 12 weist ein Knochengewinde 24 zum Einschrauben in den Knochen und einen zylinderförmigen Ansatz 25 mit einem Außengewinde auf, das mit dem Innengewinde 4 des Federelementes 1 zusammenwirkt.

Der Schraubenkopf 13 weist einen zylinderförmigen Abschnitt 27 und daran angrenzend wie der Schaft 12 einen zylinderförmigen Ansatz 26 mit einem Außengewinde auf, das mit dem Innengewinde 4' des Federelementes 1 zusammenwirkt.

Das Schraubenelement 11 ist in einen Aufnahmeteil 14 in unbelastetem Zustand schwenkbar gehalten. Das Aufnahmeteil 14 ist im wesentlichen zylindrisch ausgebildet und weist an seinem einen Ende eine axialsymmetrisch ausgerichtete erste Bohrung 15 auf, deren Durchmesser größer als der des Schafts 12 und kleiner als der des Schraubenkopfs 13 ist. Ferner weist das Aufnahmeteil 14 eine koaxiale zweite Bohrung 16 auf, die auf dem der ersten Bohrung 15 gegenüberliegenden Ende offen ist und deren Durchmesser so groß ist, daß das Schraubenelement durch das offene Ende mit dem Schaft durch die erste Bohrung 15 hindurchführbar ist, bis der Schraubenkopf 13 am Rand der ersten Bohrung 15 anliegt. Das Aufnahmeteil 14 weist eine sich vom freien Ende in Richtung der ersten Bohrung 15 erstreckende U-förmige Ausnehmung 14' auf, durch die zwei freie Schenkel 17, 18 gebildet sind. In einem Bereich angrenzend an ihr freies Ende weisen die Schenkel 17, 18 ein Innengewinde auf, welches mit einem entsprechenden Außengewinde einer Innenschraube 19 zum Fixieren eines Stabs 20 zusammenwirkt.

Es ist ferner ein Druckelement 21 zum Fixieren des Schraubenkopfs 13 in dem Aufnahmeteil 14 vorgesehen, das so ausgebildet ist, daß es an seiner dem Schraubenkopf 13 zugewandten Seite eine sphärische-Ausnehmung 22 aufweist, deren Radius im we-sentlichen gleich dem Radius des kugelsegmentförmigen Abschnitts des Schraubenkopfes 13 ist. Der Außendurchmesser des Druckelements 21 ist so gewählt, daß das Druckelement 21 in dem Aufnahmeteil 14 zu dem Schraubenkopf 13 hin verschiebbar ist. Das Druckelement 21 weist ferner eine koaxiale Bohrung 23 für den Zugriff auf eine nicht dargestellte Ausnehmung in dem Schraubenkopf 13 zum Ineingriffbringen mit einem Einschraubwerkzeug auf.

Im Betrieb wird in das Innengewinde 4 des Federelementes 1 der Schaft 12 mit seinem zylinderförmigen Ansatz 25 und in das Innengewinde 4' der Schraubenkopf 13 mit seinem zylinderförmigen Ansatz 26 eingeschraubt, um ein Schraubenelement 11 zu bilden. Danach wird das so gebildete Schraubenelement 11 mit dem Schaft 12 voran durch die zweite Öffnung in das Aufnahmeteil 14 eingeführt, bis der Schraubenkopf 13 am Rand der ersten Bohrung 15 anliegt. Anschließend wird das Druckelement 21 mit der sphärischen Ausnehmung voran durch die zweite Öffnung 16 in das Aufnahmeteil 14 eingeführt. Dann wird das Schraubenelement 11 in den Knochen bzw. Wirbel eingeschraubt. Schließlich wird der Stab 20 in das Aufnahmeteil 14 zwischen die beiden Schenkel 17 und 18 eingelegt, die Winkelstellung des Aufnahmeteils relativ zu dem Schraubenelement justiert und mit der Innenschraube 19 fixiert. Durch den elastischen Abschnitt werden Bewegungen um die Ruhelage in begrenzter Weise ermöglicht.

Die Polyaxialschraube ist nicht auf die zuvor beschriebene Ausführungsform beschränkt, sondern kann auch jede beliebige andere Polyaxialschraube mit einem wie oben beschriebenen dreiteiligen Schraubenelement sein. So kann die erste Bohrung 15 bei der in Fig 3a dargestellten Anwendung auch geringer im Durchmesser sein als der Schaft 12, wenn im Betrieb zuerst der schraubenkopf 13 mit seinem zylinderförmigen Ansatz 26 voran durch die zweite Bohrung 16 in das Aufnahmeteil 14 eingeführt wird, bevor das Federelement 1 und der Schaft 12 an den Schraubenkopf 12 angeschraubt wird. Es ist in diesem Fall ausreichend, wenn die erste Bohrung 15 einen größeren Durchmesser hat als der zylinderförmige Ansatz 26 und als der zylinderförmige Abschnitt 27. Alternativ kann der Schraubenkopf 13 auch ohne den zylinderförmigen Abschnitt 27 ausgebildet sein. Die Bohrung muss dann lediglich so groß sein, dass der Ansatz 26 hindurchführbar ist.

Das Aufnahmeteil kann aber auch derart gestaltet sein, dass das Schraubenelement von unten einführbar ist und über ein Druckelement im Aufnahmeteil verklemmt wird. Die in Fig. 3a gezeigte Bohrung 15 ist dann größer als der Durchmesser des Schraubenkopfes 13.

Die Stabfixierung ist nicht auf die in Fig. 3a gezeigte Innenschraube beschränkt, sondern es kann zusätzlich eine Außenmutter vorgesehen sein, oder jede bekannte Art der Stabfixierung kann eingesetzt werden.

Wenn das Federelement 1 wenigstens teilweise über die Knochenoberfläche hervorsteht kann das Federelement 1 Biegekräfte, sowie Zug- und Druckkräfte aufnehmen. Steht das Federelement nicht mehr über die Knochenoberfläche hervor, kann das Schraubenelement 11 trotzdem bei einer Bewegung des Knochens bzw. Wirbels etwas nachgeben. Damit wird verhindert, dass ungünstige Spannungen auftreten.

Fig. 3b zeigt eine Weiterbildung des Federlements 1 von Fig. 3a. Das Federelement 640 gemäß Fig. 3b weist in seinem Inneren einen Kern 641 auf. Der Kern 641 ist an seinen Enden 642 zylindrisch ausgebildet mit einem Durchmesser, der so bemessen ist, daß der Kern in den Hohlraum des elastischen Abschnitts 640 einschiebbar ist. Die zylindrischen Abschnitte 642 und der elastische Abschnitt 640 weisen querverlaufende Bohrungen auf, in die Stifte 643 zum Befestigen des Kerns geschoben sind. Zwischen den zylindrischen Enden weist der Kern einen Abschnitt 644 mit im wesentlichen rechteckigem Querschnitt auf, wie insbesondere aus Fig. 3c ersichtlich ist. In einer Abwandlung hat der Kern einen anderen Querschnitt, wie z.B. oval oder asymmetrisch. Der Kern erlaubt die Einstellung der Biegesteifigkeit und/oder der Torsionssteifigkeit des elastischen Abschnitts. Die Steifigkeit des elastischen Abschnitts gegenüber einer Biegung in einer bestimmten Richtung hängt von der Orientierung des Kerns innerhalb des elastischen Abschnitts ab. Vorzugsweise ist der Kern aus einem Material mit geringerer Steifigkeit verglichen mit dem Material des elastischen Abschnitts ausgebildet. Die gezeigte Befestigung des Kerns ist nur beispielhaft dargestellt.

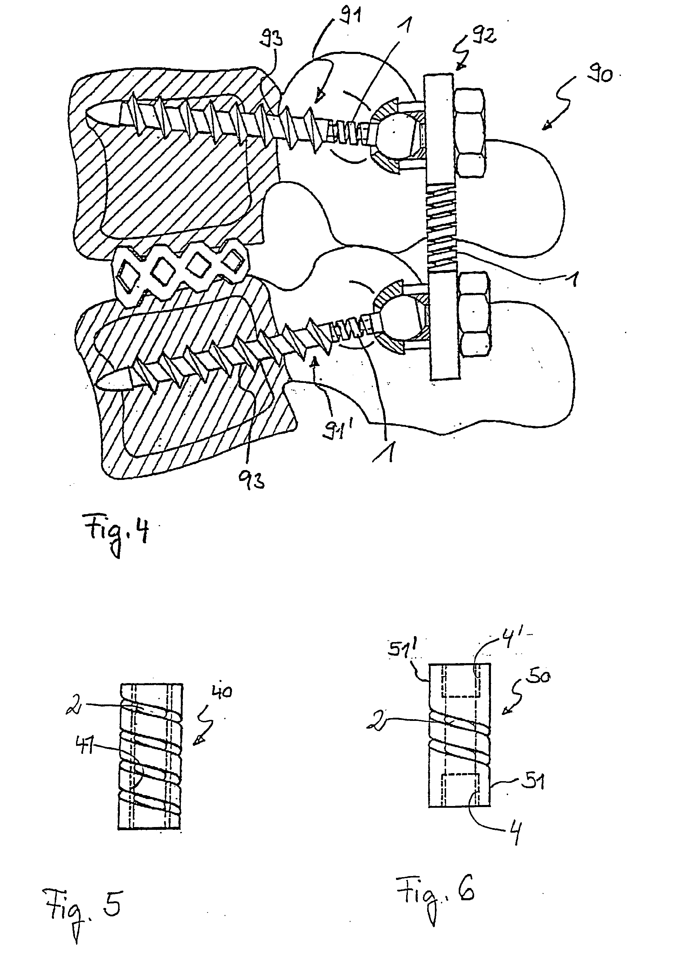

In Fig. 4 ist eine Stabilisierungsvorrichtung 90 für die Wirbelsäule dargestellt, wobei zwei Knochenverankerungselemente 91, 91' mit Schraubenelementen 93 und ein elastisches stabförmiges Element 92 jeweils mit einem Federelement 1 zur Verbindung der beiden Knochenverankerungselemente verwendet wird. Durch die Mehrteiligkeit des elastischen stabförmigen Elementes und des Schraubenelementes ist es möglich, durch die Kombination von nur wenigen Grundelementen Stabilisierungsvorrichtungen 90 mit den unterschiedlichsten Eigenschaften zu erhalten. Die Stabilisierungsvorrichtung muß nicht notwendigerweise Knochenverankerungselemente mit einem Federelement und ein stabförmiges Element mit dem Federelement beinhalten. Je nach Anwendungsgebiet ist es auch möglich, nur ein stabförmiges Element mit Federelement und Knochenverankerungselemente mit starren Schraubenelementen vorzusehen.

In Fig. 5 ist ein zweites Federelement 40 dargestellt. Das zweite Federelement 40 unterscheidet sich von dem ersten Federelement 1 nur darin, dass anstelle der beiden Innengewinde 4, 4' ein über die ganze Länge des Federelementes ausgebildetes Innengewinde 41 vorgesehen ist.

In Fig. 6 ist ein drittes Federelement 50 dargestellt. Es weist im Unterschied zum ersten oder zweiten Federelement starre Endabschnitte 51 und 51' bzw. eine im Unterschied zu den vorher beschriebenen Federelementen verringerte Anzahl von Spiralwindungen auf. Dadurch lässt sich die Elastizität des Federelementes unabhängig von der Länge des Federelementes gestalten.

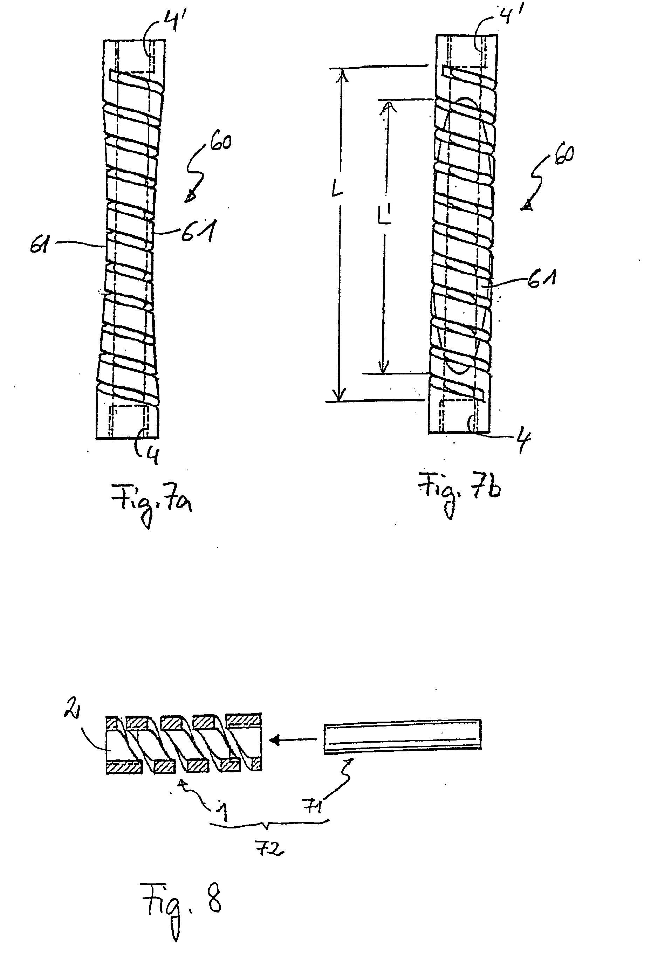

In den Figuren 7a und 7b ist ein viertes Federelement 60 dargestellt, das im Unterschied zu den vorgehenden Federelementen zwar um 180° gegeneinander versetzte konkav zur Mittenachse hin angeformte Bereiche 61 aufweist. Die Länge L' der Bereiche 61 in Richtung der Mittenachse ist maximal gleich der Länge L der Spirale und der Krümmungsradius der angeformten Bereiche 61 ist derart, daß die Windungen der Schraubenfeder nicht durchbrochen sind. Durch diese Ausbildung ist das Federelement in einer Richtung senkrecht zur Mittenachse tailliert ausgebildet und hat somit eine geringere Steifigkeit in dieser Richtung. Damit hat das Federelement eine orientierte Steifigkeit, die für bestimmte Anwendungen zweckmäßig ist.

Ein in Fig. 8 dargestelltes fünftes Federelement 72 weist zusätzlich zu dem ersten Federelement 1 einen in die Bohrung eingeschobenen stabförmigen Kern 71 auf. Der Kern kann einerseits als Anschlag bei auf das Federelement 72 ausgeübten Druckkräften dienen. Andererseits kann mit dem Kern 71 die Steifigkeit des Federelementes 72 in bezug auf Biegekräfte erhöht werden.

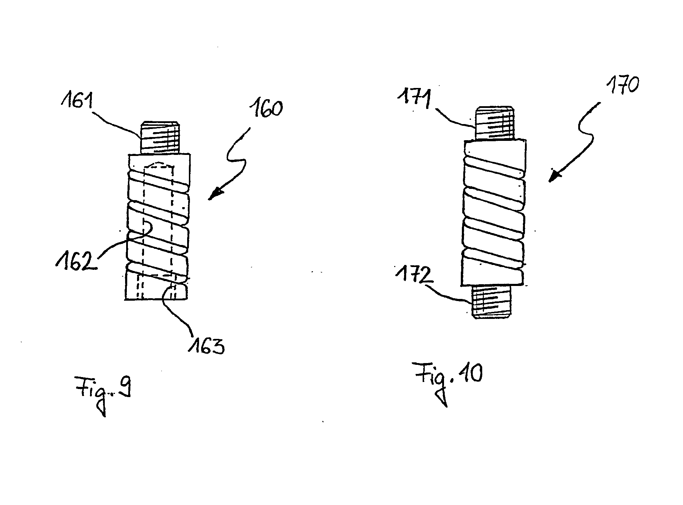

Ein in Fig. 9 dargestelltes sechstes Federelement 160 weist an seinem einen Ende, anstelle einer Bohrung mit einem Innengewinde wie bei dem ersten Federelement, einen zylindrischen Ansatz 161 mit einem Außengewinde auf. Entsprechend ist das mit diesem Ende des Federelements zu verbindende Element mit einer Bohrung mit einem entsprechenden Innengewinde ausgebildet. Das andere Ende des Federelements ist mit einer Sackbohrung 162 versehen, in der an das Ende des Federelementes angrenzend wie bei den zuvor beschriebenen Federelementen ein Innengewinde 163 ausgebildet ist.

Ein in Fig. 10 dargestelltes siebtes Federelement 170 weist an seinen beiden Enden jeweils einen zylinderförmigen Ansatz 171, 172 mit einem Außengewinde auf.

In einer Abwandlung der ersten bis fünften Federelemente weist das Federelement keine durchgehende Bohrung auf.

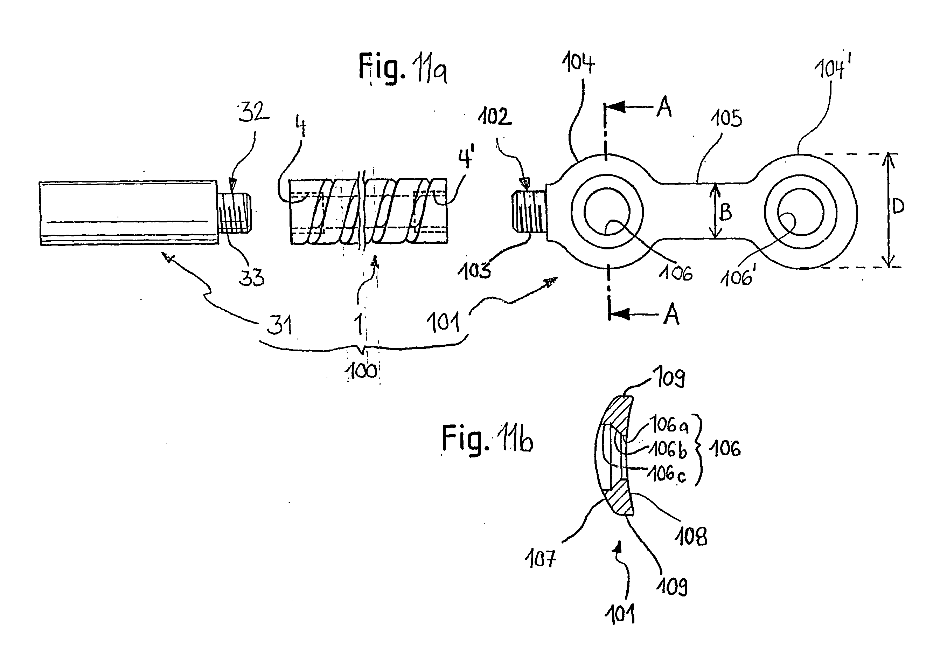

Als weitere Anwendung für das Federelement 1 ist in Fig. 11a die Explosionsdarstellung eines Verbindungselementes 100 zu sehen, das aus einem stabförmigen Element 31, einem Federelement 1 und einer Platte 101 besteht. Das stabförmige Element 31 weist einen zylindrischen Ansatz 32 mit einem Außengewinde 33 zum Einschrauben in das an das eine Ende des Federelementes 1 angrenzende Innengewinde 4 auf. Ebenso weist die Platte 101 einen zylindrischen Ansatz 102 mit einem Außengewinde 103 zum Einschrauben in das an das andere Ende des Federelementes 1 angrenzende Innengewinde 4' auf. Die Platte besteht aus zwei in der Draufsicht kreisförmigen Abschnitten 104, 104', die über einen Steg 105 miteinander verbunden sind. Die Breite B des Steges 105 ist geringer als der Durchmesser D der kreisförmigen Abschnitte 104, 104'. Koaxial zu den kreisförmigen Abschnitten sind zwei Bohrungen 106, 106' für Senkschrauben durch die Platte vorgesehen. Wie in Fig. 11b zu sehen weist die erste Seite 107 der Platte eine konkave Krümmung auf während die zweite Seite 108 der Platte eine konvexe Krümmung zum Anliegen dieser Seite an einen Knochen aufweist. Durch die unterschiedlichen Krümmungsradien der beiden seiten 107, 108 der Platte 101 verjüngt sich die Platte 101 zu den seitlichen Rändern 109 hin. Dadurch kann die Platte stabil und gleichzeitig raumsparend sein. Die Bohrungen 106, 106' weisen wie in Fig.

11b zu sehen an die zweite Seite 108 angrenzend eine Öffnung 106a, daran angrenzend einen konisch geformten ersten Abschnitt 106b und einen an den ersten Abschnitt und die erste Seite 107 angrenzenden zweiten Abschnitt 106c auf. Durch diese Form der Bohrungen 106, 106' sind diese zum Aufnehmen von Senkschrauben geeignet ausgebildet. Die Form der Bohrungen 106, 106' kann auch von der zuvor beschriebenen Form abweichen, soweit sie zur Aufnahme einer Senkschraube geeignet ist.

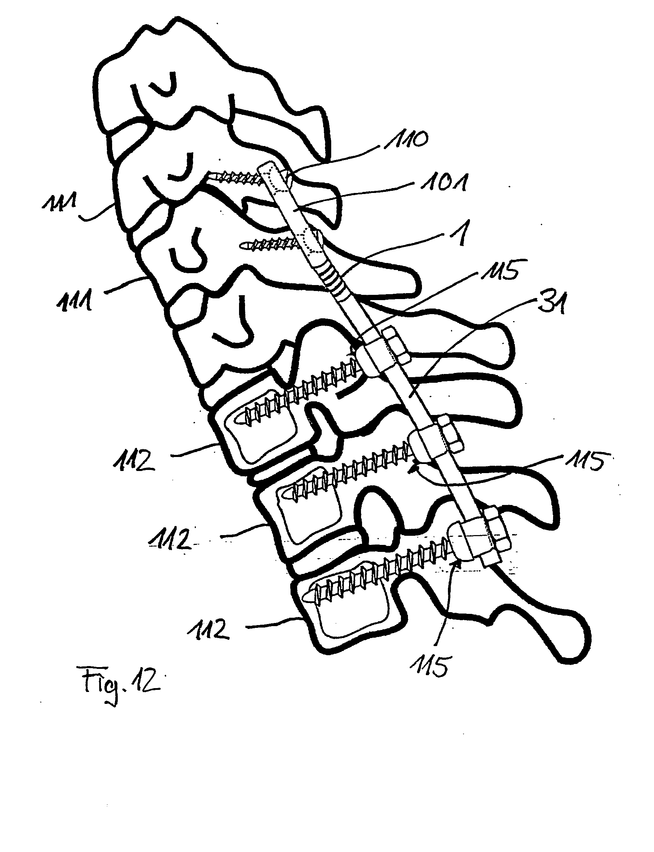

In Fig. 12 ist eine Anwendung für das Verbindungselement 100 aus Fig. 11a, bei dem die Platte 101 von der posterioren Seite mit zwei Knochenschrauben 110 an zwei Wirbeln 111 der Halswirbelsäule fixiert ist, und bei dem das mit der Platte über ein Federelement 1 verbundene stabförmige Element 31 mit drei Knochenverankerungselementen 115 in Wirbeln 112 der Brustwirbelsäule verankert ist.

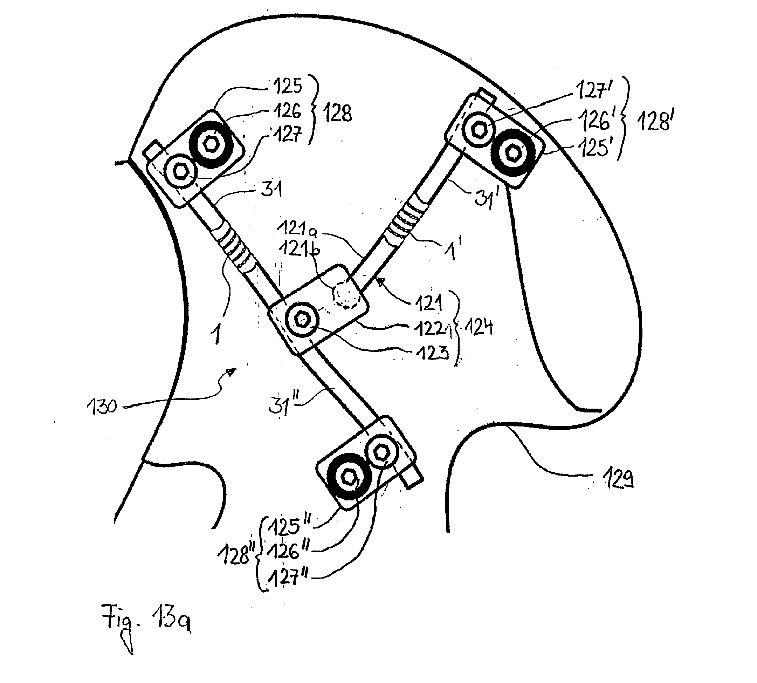

Eine weitere Anwendung, bei dem das Federelement 1 in einer dynamischen Beckenstabilisierüngsseinrichtung 130 verwendet wird, ist in Fig. 13a dargestellt. Die dynamische Beckenstabilisierungseinrichtung besteht aus Knochenverankerungselementen 128, 128', 128", die mit stabförmigen Elementen 31, 31', 31" und Federelementen 1, 1' miteinander verbunden sind.

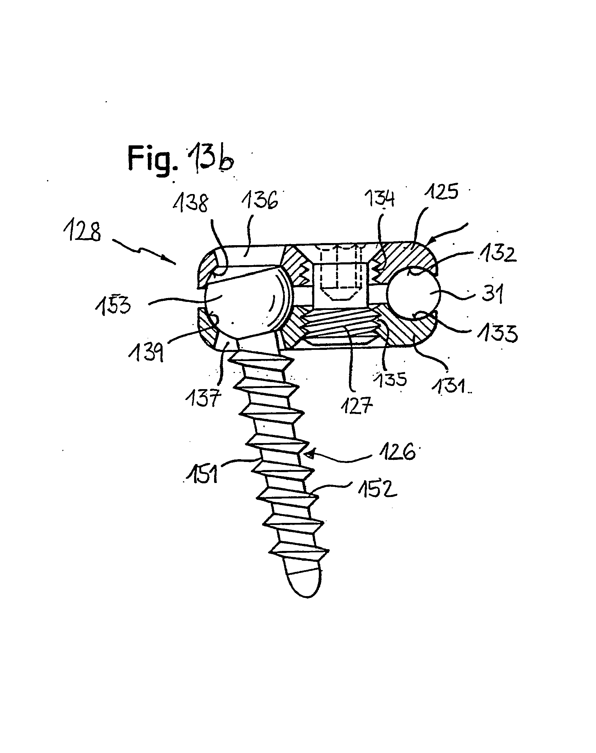

Das in Fig. 13b dargestellte Knochenverankerungselement 128 besteht wie die beiden anderen Knochenverankerungselemente 128', 128" aus zwei Hälften 125, 131, die mit einer in ein Gewinde 134 in der ersten Hälfte 125 und in eine Gewinde 135 in der zweiten Hälfte 131 eingreifenden Schraube 127 miteinander verschraubt sind. In der Draufsicht in Fig. 13a ist davon nur die eine obere Hälfte 125 sichtbar. Zwischen den beiden zuvor erwähnten Hälften 125, 131 ist das stabförmige Element 31 in einer Ausnehmung 132 in der ersten Hälfte 125 und in einer Ausnehmung 133 in der zweiten Hälfte 131 zwischen den beiden Hälften eingeklemmt, sodass das Knochenverankerungselement 128 fest mit dem stabförmigen Element 31 verbunden ist. Weiter sind in den beiden Hälften 125, 131 jeweils eine Bohrung 136 bzw. 137 vorgesehen, die im montierten Zustand Koaxial ausgerichtet sind. An die Bohrung 136 angrenzend ist eine sphärische Ausnehmung 138 und an die Bohrung 137 angrenzend ist eine sphärische Ausnehmung 139 ausgebildet, die der Aufnahme einer Knochenschraube 126 dienen. Die Knochenschraube 126 weist einen schaftförmigen Abschnitt 151 mit einem Außengewinde 152 zum Einschrauben in den Knochen und einen kugelsegmentförmigen Kopfabschnitt 153 mit einem Radius der im wesentlichen gleich dem Radius der sphärischen Ausnehmungen 138, 139 ist.

Das Verbindungselment 124 besteht wie das Knochenverankerungselement aus zwei Hälften 122, von denen in der Draufsicht nach Fig. 13a nur eine zu sehen ist. Zwischen diese beiden zuvor erwähnten Hälften 122 ist das stabförmige Element 31 in einer Ausnehmung geführt eingeklemmt, sodass das Verbindungselement 124 fest mit dem stabförmigen Element 31 verbunden ist.

Das Stabelement 121 besteht aus einem kugelförmigen Kopfabschnitt 121b und einem Schaftabschnitt 121a. Der Kopfabschnitt 121b wird zwischen den beiden Hälften 122 in einer nicht dargestellten Ausnehmung festgeklemmt und ist so in einer bestimmten Schwenkstellung fixierbar mit den beiden Hälften 122 verbunden. An seinem dem Kopfabschnitt 121b gegenüberliegenden Ende weist der Schaftabschnitt 121a einen zylinderförmigen Ansatz (nicht dargestellt) mit einem Außengewinde auf, das in das Innengewinde (nicht dargestellt) des Federelementes 1' eingeschraubt ist.

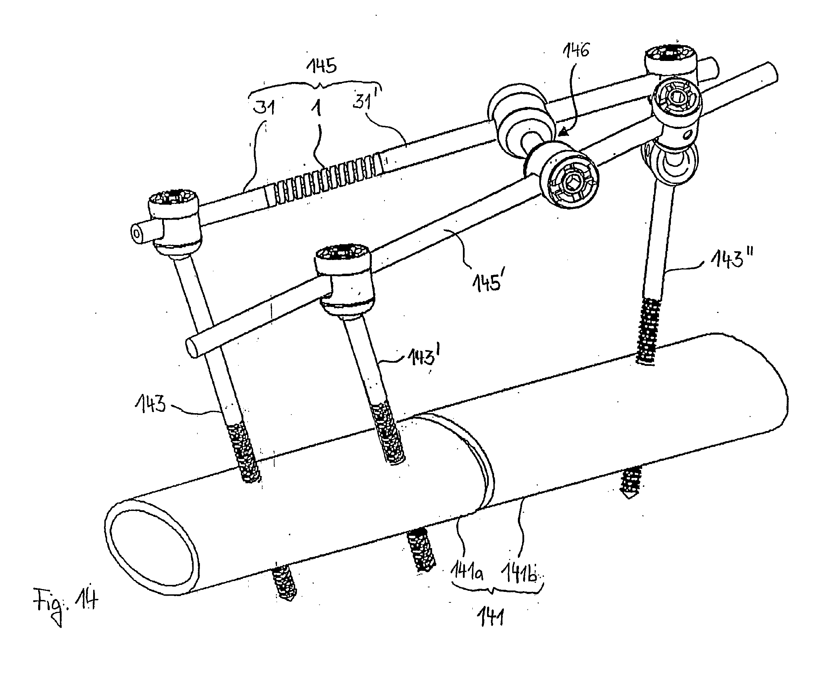

In Fig. 14 ist eine weitere Anwendung für das Federelement 1 dargestellt. Das Federelement 1 ist hier Teil eines Fixateur Externe zum Stabilisieren eines aus zwei Teilen 141a und 141b bestehenden Knochens 141.

In das erste Teil 141a des Knochens 141 sind eine erste und eine zweite Schanzschraube 143, 143' und in den zweiten Teil des Knochens 141b des Knochens 141 ist eine dritte Schanzschraube 143" eingeschraubt. Die erste Schanzschraube 143 und die zweite Schanzschraube 143' sind in an sich bekannter Art und Weise über einen ersten Stab 145 bzw. einen zweiten Stab 145' mit der dritten Schanzschraube 143" verbunden. Der erste und der zweite Stab 145, 145' sind zusätzlich mit einem hier nicht näher beschriebenen Kopplungselement 146 in an sich bekannter Art und Weise fest miteinander verbunden.

Der erste Stab 145 ist dreiteilig aus zwei wie weiter oben mit Bezug auf Fig. 2 beschriebenen stabförmigen Elementen 31, 31' und einem Federelement 1 ausgebildet. Dabei ist das erste stabförmige Element über eine Schraubverbindung fest mit dem einen Ende des Federelementes 1 und das zweite stabförmige Element über eine Schraubverbindung fest mit dem anderen Ende des Federelementes 1 verbunden.

Durch die dynamische Stabilisierung des Knochens 141 werden kleinere Bewegungen der beiden Knochenenteile 141a und 141b untereinander erlaubt. Diese kleineren Bewegungen führen zu einer wünschenswerten Stimulation für das Zusammenwachsen der beiden Knochenteile 141a, 141b.

Je nach Anwendungsgebiet kann auch eine Schanzschraube des Fixateur Externe ein derartiges Federelement als Teil seines Schaftes aufweisen.

Als weitere Anwendungen für das Federelement gilt zum Beispiel der Einsatz in einer Knochenschraube, die nur einen Schaft mit einem Knochengewinde und eine Spitze sowie einem Kopf aufweist, wie sie üblicherweise zur Verbindung mit Stabilisierungsplatten verwendet werden. Eine weitere Anwendung des Federelements ist der Einsatz in einer Monoaxial-Knochenschraube zum Verbinden mit einem Stab.

Zur Herstellung eines Federelementes 1 mittels Fräsen geht man von einem Zylinder mit einem vorbestimmten Aussendurchmesser aus einem biokompatiblen Material, wie z.B. Titan, aus und fräst mit einem dünnen Scheibenfräser entlang einer Spirale, deren Hauptachse kollinear zu der Hauptachse des Zylinders ist eine Ausnehmung 3. Anschließend wird entlang der Hauptachse des Zylinders über die gesamte Länge des Zylinders eine Bohrung 2 derart gebildet, dass die spiralförmige Ausnehmung 3 in die Bohrung 2 mündet. Für die Stabilität des Federelementes 1 ist der Auslauf der Spirale am Übergang zwischen dem Spiralabschnittes und dem endseitigen Abschnitt des Federelementes von größter Bedeutung. Daher ist es notwendig, mit einem Fingerfräser den Auslauf der Spirale an beiden Enden der Spirale derart nachzubearbeiten, dass die scharfe Kante an der Innenseite der Bohrung entfernt wird. Dazu wird der Auslauf mit einem Fingerfräser in einem Winkel tangential zur Spiralkontur gefräst. Im Anschluß daran wird das Bauteil innen und außen entgratet. Schließlich wird in den zwei Endabschnitten der Bohrung 2 jeweils ein Innengewinde 4, 4' gebildet.

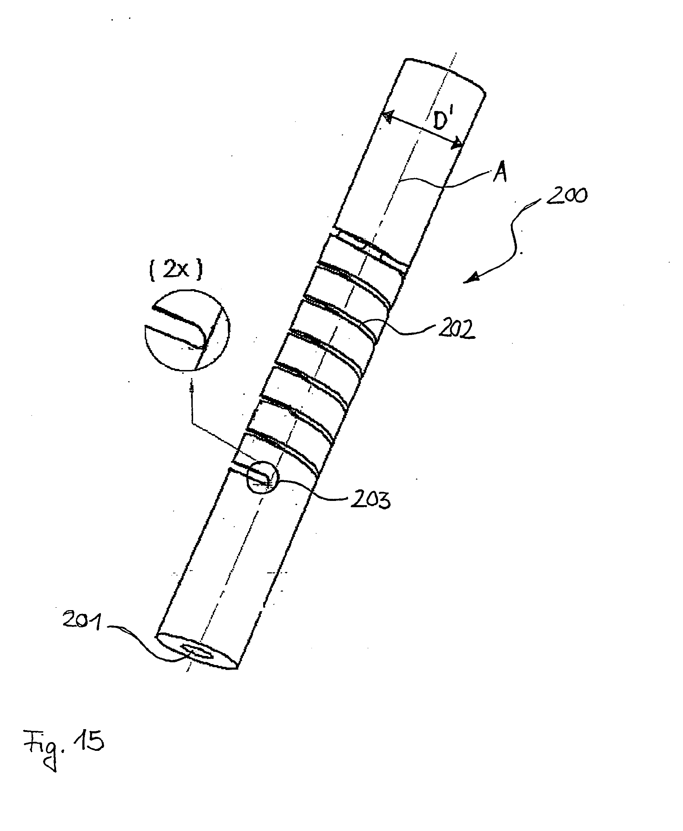

Alternativ zum Fräsen wird das Federelement 200 mit Drahterodieren, Laserbearbeitung oder Wasserstrahlbearbeitung aus dem zylinderförmigen Körper hergestellt. Hierbei wird wie in der erfindungsgemäßen Ausführungsform gemäß Fig. 15 gezeigt wiederum von einem Zylinder mit vorbestimmten Aussendurchmesser D' ausgegangen, in den dann im nächsten Schritt eine Bohrung 201 entlang der Hauptachse A über die gesamte Länge des Zylinderkörpers gebildet wird. Dann wird in die Wand des so gebildeten Hohlzylinders entlang der Hauptachse die Wand entlang einer Spirale 202 je nach Wandstärke des Hohlzylinders durch eines der oben angesprochenen Verfahren geschnitten. Der Auslauf 203 der Spirale 202 wird als Viertelkreis gebildet, sodass eine Nachbearbeitung des Auslaufs 203 in einem weiteren Arbeitsschritt gegenüber dem Fräsen entfällt. Auch ein Entgraten ist bei diesem Herstellungsverfahren nicht notwendig. Die Viertelkreisform des Auslaufs hält Belastungsspitzen in dem Material im Betrieb gering.

Schließlich wird wie bei dem Herstellungsverfahren mittels Fräsen in den zwei Endabschnitten der Bohrung 2 jeweils ein Innengewinde 4, 4' gebildet.

In einer Abwandlung wird bei den obigen Verfahren anstelle zumindest eines der Innengewindes 4, 4' zu Beginn des Verfahrens durch Drehen ein zylinderförmiger Ansatz mit einem Aussengewinde gebildet. In diesem Fall muß der Durchmesser der Bohrung kleiner als der Durchmesser des zylinderförmigen Ansatzes sein.

In einer weiteren Abwandlung des Herstellungsverfahrens wird das Federelement entsprechend dem sechsten und siebten Federelement ohne eine durchgehende Bohrung hergestellt.

Als Materialien für das Federelement, den Kern oder der damit verbundenen Elemente sind körperfreundliche Materialien, wie z.B. ein körperfreundliches Metall, wie Titan oder ein körperfreundlicher Kunststoff möglich. Auch die Verwendung einer Formgedächtnislegierung mit bekannten superelastischen Eigenschaften, z.B. Nitinol, ist möglich.

## Patentansprüche

1. Elastisches Element (200) zur Verwendung in einer Stabilisierungseinrichtung (130) für Knochen oder Wirbel,
welches als ein rohrförmiger, im wesentlichen zylindrischer Körper mit einer spiralförmigen Ausnehmung (202) in seiner Wandung entlang seiner Hauptachse (A) mit einem ersten Ende und einem diesem gegenüberliegenden zweiten Ende und einer sich von dem ersten zum zweiten Ende erstreckenden koaxialen Bohrung (201) ausgebildet ist, wobei die Ausnehmung (202) in radialer Richtung in die Bohrung (201) des Körpers mündet, der an seinen gegenüberliegenden Enden jeweils entweder ein Innengewinde (4, 4', 41, 163) oder einen zylindrischen Ansatz mit einem Außengewinde (161, 171, 172) zum Verbinden mit einem Schaft und/oder einem Kopf einer Knochenschraube oder zum Verbinden mit einem Stababschnitt oder einer Platte aufweist, wobei
in der Bohrung (202) ein Kern (71, 644) vorgesehen ist, der derart gestaltet ist, dass die Biegesteifigkeit des elastischen Elements erhöht wird, **dadurch** gakennzeichnet, dass
die spiralförmige Ausnehmung _(202) einen Auslauf (203) aufweist, wobei der Auslauf zur Geringhaltung von Belastungsspitzen in dem Material des Körpers als Viertelkreis ausgebildet ist.

2. Elastisches Element nach Anspruch 1, **dadurch gekennzeichnet, dass** an beiden Enden ein Innengewinde (4, 4', 41, 163) vorgesehen ist.

3. Elastisches Element nach Anspruch 1, **dadurch gekennzeichnet, dass** an beiden Enden ein zylindrischer Ansatz mit einem Außengewinde (171, 172) vorgesehen ist.

4. Elastisches Element nach Anspruch 1, **dadurch gekennzeichnet, dass** an einem Ende ein Innengewinde (163) und am anderen Ende ein zylindrischer Ansatz mit Außengewinde (161) vorgesehen ist.

5. Elastisches Element nach einem der Ansprüche 1 bis 4, **dadurch** gekenntzeichnet, dass das Element als Schraubenfeder ausgebildet ist.

## Claims

1. Elastic element (200) for use in a stabilization device (130) for bones or vertebrae,
which is formed as a tubular, substantially cylindrical body having a helical recess (202) formed in its wall along its main axis (A), a first end and a second end opposite the first end and a coaxial bore (201) extending from the first end to the second end,
wherein the recess (202) opens into the bore (201) of the body in a radial direction, which has at its opposite ends either an inner thread (4, 4', 41, 163) or a cylindrical boss having an outer thread (161, 171, 172) for connection with a shaft and/or a head of a bone screw, or for connection with a rod portion or a plate, wherein
a core (71, 644) is provided within the bore (202), which is formed such as to increase a bending stiffness of the elastic element,
**characterized in that**
the helical recess (202) has a runout portion (203), the runout portion (203) being formed as a quarter circle to keep reduced load maxima created in the material of the body.

2. Elastic element according to claim 1, **characterized in that** an inner thread (4, 4', 41, 163) is provided at both ends.

3. Elastic element according to claim 1, **characterized in that** a cylindrical boss having an outer thread (171, 172) is provided at both ends.

4. Elastic element according to claim 1, **characterized in that** an inner thread (163) is provided at one end, and a cylindrical boss having an outer thread (161) is provided at the other end.

5. Elastic element according to one of claims 1-4, **characterized in that** the element is formed as a helical spring.

## Revendications

1. Elément élastique (200) pour utilisation dans un dispositif de stabilisation (130) pour des os ou des vertèbres,
réalisé sous forme de corps sensiblement cylindrique, tubulaire, avec un évidement (202) à forme spirale dans sa paroi, le long de son axe principal (A), avec une première extrémité et une deuxième extrémité, opposée à celle-ci, et un perçage (201) coaxial, s'étendant de la première à la deuxième extrémité, l'évidement (202) débouchant en direction radiale dans le perçage (201) du corps, présentant, à chacune de ses extrémités opposées, soit un filetage intérieur (4, 4', 41, 163), soit un appendice cylindrique avec un filetage extérieur (161, 171, 172), pour la liaison à une tige et/ou une tête d'une vis à os ou pour la liaison à un tronçon de barre ou à une plaque, où,
dans le perçage (202), est prévu un noyau (71, 644) configuré de manière que la rigidité en flexion de l'élément élastique soit augmentée, **caractérisé en ce que**
l'évidement (202) à forme spirale présente un débouché (203), le débouché étant configuré en quart de cercle, pour le maintien à une faible valeur des pics de charge dans le matériau du corps.

2. Elément élastique selon la revendication 1, **caractérisé en ce qu'**un filetage intérieur (4, 4', 41, 163) est prévu aux deux extrémités.

3. Elément élastique selon la revendication 1, **caractérisé en ce qu'**un appendice cylindrique muni d'un filetage extérieur (171, 172) est prévu aux deux extrémités.

4. Elément élastique selon la revendication 1, **caractérisé en ce qu'**un filetage intérieur (163) est prévu à une extrémité et un appendice cylindrique muni d'un filetage extérieur (161) est prévu à l'autre extrémité.

5. Elément élastique selon l'une des revendications 1 à 4, **caractérisé en ce que** l'élément est réalisé sous forme de ressort hélicoïdal.
